# EUROPÄISCHE PATENTSCHRIFT

(11) **EP 4 239 327 B1**
(45) Veröffentlichungstag und Bekanntmachung des Hinweises auf die Patenterteilung: **28.10.2026**
(21) Anmeldenummer: 23158812.0
(22) Anmeldetag: 27.02.2023
(51) Int. Cl.: G01N 27/02

(54) **BIOFILMSENSOR UND VERFAHREN ZUR BIOFILMDETEKTION**
BIOFILM SENSOR AND METHOD FOR BIOFILM DETECTION
CAPTEUR DE BIOFILM ET PROCÉDÉ DE DÉTECTION DE BIOFILM

(30) Priorität: 02.03.2022 DE 102022104917; 04.04.2022 DE 102022108009
(43) Veröffentlichungstag der Anmeldung: 06.09.2023
(73) Patentinhaber: Schellbach, Winfried, 07546 Gera (DE)
(72) Erfinder: Schellbach, Winfried, 07546 Gera (DE)
(74) Vertreter: Meissner Bolte Partnerschaft mbB

(56) Entgegenhaltungen:
- DE-A1- 102011 082 072
- DE-A1- 102013 214 392
- DE-A1- 102017 106 704
- DE-A1- 19 844 329
- KR-B1- 100 949 311

## Beschreibung

Die Erfindung betrifft einen Biofilmsensor nach Anspruch 1 und ein Verfahren zur Biofilmdetektion nach Anspruch 6.

Die Bildung und Ablagerung von Biofilmen in flüssigkeitsgefüllten Systemen wird in umfassenden Artikeln, Berichten und Dokumentationen ausführlich beschrieben. Biofilme stellen dabei ein großes industrielles und gesellschaftliches Problem dar. Sie bergen ein besonderes Gefährdungspotential für Korrosionen, Schichtenbildungen mit Energieverlusten in Anlagen und nicht zuletzt auch Gesundheitsgefahren.

Biofilme entstehen immer in wässrigen Systemen. In diesen leben unterschiedlichste Mikroorganismen, welche eine extrazelluläre polymere Schicht ausbilden und darunter einen eigenen Lebensraum der Mikroorganismen schützen. Unabhängig der Materialwandungen und ihres Alters werden solche Biofilme gebildet. Entsprechend den Umgebungsbedingungen wächst der Biofilm in der Fläche und Dicke. Desinfektionsmaßnahmen wie Chlorung, Heißwasser- oder Clean-in-place-Prozesse (CIP) können weitestgehend die Biofilmbildung beseitigen oder stoppen, jedoch werden nach kurzer Zeit wieder alte Biofilme aktiviert und neue gebildet. Ein Problem ist dabei die kontinuierliche messtechnische Erfassung, Kalibrierung und Überwachung von Biofilmen in flüssigkeitsgefüllten Systemen.

So gibt es akustische Verfahren, die in Edelstahlrohrleitungen mit DN 40 bis DN 50 Schichtdicken von Biofilmen in der Stärke von 50 - 5000 µm erfassen und kontrollieren können.

Weiterhin sind Biosensoren bekannt, welche nach den Prinzipien einer elektrochemischen Sonde arbeiten, deren Betrieb auf der kathodischen Depolarisation beruht, die durch das Biofilmwachstum induziert wird, oder eine Sonde, die mit Differentialturbidimetrie,Lichtstreuung, Wärmeübertragung, Druckabfall, Echtzeitmessung von Stoffwechselprodukten, Bildanalyse, Strahlungssignalen oder einem elektrischen Signal oder Vibrationssignalen arbeitet.

Es sind auch optische Biofilmsensoren zur Bestimmung biologischer Eigenschaften eines Biofilms auf einem Siedlungssubstrat bekannt. Diese enthalten insbesondere folgende Komponenten: einen Sensorkopf, einer im Sensorkopf angeordneten, ein definiertes Emissionsspektrum ausstrahlenden Lichtquelle und wenigstens einen Detektor zum Detektieren des vom Biofilm emittierten Lichts. Der Sensorkopf ist dadurch gekennzeichnet, dass eine Mehrzahl lichtleitender Glasfasern unterhalb des Siedlungssubstrats ringförmig um die zentral im Sensorkopf angeordnete Lichtquelle angeordnet sind, wobei die lichtleitenden Glasfasern wenigstens in Bezug auf eine räumliche Achse in einem Anstellwinkel schwenkbar eingerichtet sind. Dokument KR 100 949 311 B1 offenbart einen Biofilmsensor mit zwei plattenförmigen Elektroden und röhrenförmigen Glasgehäuse.

Es werden auch Biofilmsensoren mit einem Verfahren zur Bestimmung der Biofilmbildung an Oberflächen in Fluiden beschrieben, bei denen ein mikrofluidisches, multiparametrisches Sensorsystem zur Bestimmung der Biofilmbildung verwendet wird, wobei die Bestimmung verschiedener Parameter unter standardisierten Bedingungen erfolgt, um eine genaue Aussage zur Biofilmbildung an Oberflächen treffen zu können.

Die vorgenannten Vorrichtungen und Verfahren zur Biofilmdetektion erweisen sich oft gerätemäßig als aufwändig sowie kostenintensiv. Es besteht somit die Aufgabe, eine vielseitig einsetzbare und aufwandsarme Lösung anzugeben, mit der kontinuierlich und wirtschaftlich das Wachstum von Biofilmen verfolgt und die Wirksamkeit eingesetzter Wasserbehandlungsmaßnahmen zur Biofilmreduktion überprüft werden kann.

Die Aufgabe wird mit dem beanspruchten Biofilmsenor nach Anspruch 1 und dem mit dem Biofilmsensor ausgeführten Verfahren zur Biofilmdetektion nach Anspruch 6 gelöst.

Der erfindungsgemäße Biofilmsensor weist eine Elektrodenanordnung auf. Die Elektrodenanordnung besteht aus einer mit einer Flüssigkeit durchströmbaren elektrisch kontaktierten rohrförmigen Keramikelektrode aus einem elektrisch lei fähigen Keramikmaterial und einer im Lumen, d.h. im röhrenförmigen Hohlraum der Keramikelektrode verlaufenden elektrisch kontaktierten Stabelektrode. An der rohrförmigen Keramikelektrode ist ein Probeneingangsanschluss für ein Probeneingangsrohr und ein Probenausgangsanschluss für ein Probenausgangsrohr angeordnet. Die rohrförmige Keramikelektrode und die innere Stabelektrode sind dabei elektrisch isolierend voneinander montiert und kontaktiert.

Dieser Aufbau bezweckt ein Durchströmen der rohrförmigen Keramikelektrode mit mikrobiologisch belasteten und elektrisch leitendem Wasser. Hierbei kommt es zur Biofilmablagerung auf dem Innenbereich der durchströmten Keramikelektrode. Der abgelagerte Biofilm beeinflusst die elektrischen Eigenschaften der Elektrodenanordnung. Diese können messtechnisch nachgewiesen werden.

Bei einer vorteilhaften Ausführungsform ist das elektrisch leitfähige unterstöchiometrische Keramikmaterial der rohrförmigen Keramikelektrode ein wasserdichtes und druckbeständiges elektrisch leitfähiges unterstöchiometrisches Titandioxidmaterial. Dieses zeichnet sich dadurch aus, dass im Gitter der Keramik kationische oder anionische Fehlstellen vorhanden sind, die als elektrischer Ladungs-träger dienen.

Eine derartige Keramik erweist sich als Substrat als besonders gut für eine Biofilmablagerung geeignet. Es handelt sich hierbei nicht um metallisches Titan, sondern um ein Titanoxid. Die biologische Besiedelbarkeit von Titandioxid-Oberflächen ist aus Untersuchungen über deren Biokompatibilität dokumentiert.

Wie beispielsweise den Publikationen "Modification of Titanium Implant and Titanium Dioxide for Bone Tissue Engineering" Ahn et. al., Adv. Exp. Med. Biol. 2018; 1077: 355-368 oder auch Abdulhameed et. Al. "Titanium dioxide dental implants surfaces related oxidative stress in bode remodeling: a systematic review" PeerJ. 2022, Mar. 3; 10: e 12951 zu entnehmen ist, erweisen sich Titanoxide als ein hervorragendes Substrat zur Besiedelung von biologischem Gewebe, insbesondere auch als Teil von Implantaten. Daher sind derartige Materialien zur Besiedelung mit Biofilmen zweckmäßig einsetzbar.

Bei der erfindungsgemäßen Ausführungsform kommen nun die genannten unterstöchiometrischen Titandioxidmaterialien zum Einsatz, die neben der biologischen Besiedelbarkeit eine elektrische Leitfähigkeit aufweisen.

Die rohrförmige Keramikelektrode ist elektrisch isolierend bezüglich der inneren Stabelektrode montiert und kontaktiert. Der Stromfluss zwischen der Keramikelektrode und der Stabelektrode verläuft somit ausschließlich durch die im Lumen, d.h. im röhrenförmigen Hohlraum der Keramikelektrode befindliche Flüssigkeit, insbesondere Wasser.

Die Stabelektrode im röhrenförmigen Hohlraum der Keramikelektrode besteht aus einem korrosionsbeständigen metallischen Material, insbesondere aus einem Edelstahl.

Außerdem ist eine zwischen die Keramikelektrode und die Stabelektrode schaltbare Messanordnung zur Bestimmung eines frequenzabhängigen komplexen elektrischen Widerstandes der mit der Flüssigkeit durchströmten Elektrodenanordnung vorgesehen.

Es hat sich gezeigt, dass ein Biofilmbewuchs im Bereich der Elektrodenanordnung besonders deutlich im komplexen elektrischen Widerstand der Elektrodenanordnung, insbesondere in dessen frequenzabhängigem Imaginärteil und dessen Änderung, nachweisbar ist.

Bei einer weiteren Ausgestaltung ist eine zwischen die Keramikelektrode und die Stabelektrode schalt- und parametrisierbare Gleichspannungsquelle zum Anlegen einer Gleichspannung und zum Bewirken einer Elektrolyse vorgesehen, die einen Biofilm in der Elektrodenanordnung reduzieren und beseitigen kann. Diese ermöglicht ein Reinigen des Biofilmsensors und dessen Rekalibrierung durch eine Wiederherstellung in den Ausgangszustand.

Das Verfahren zur Biofilmdetektion in einer flüssigkeitsgefüllten Umgebung erfolgt mit einem Detektionsmodus mit folgenden Verfahrensschritten:
Es wird ein Biofilmsensor mit einer Elektrodenanordnung aus einer rohrförmigen Keramikelektrode und einer im Lumen, d.h. im röhrenförmigen Hohlraum der Keramikelektrode angeordneten Stabelektrode verwendet.

Die Elektrodenanordnung wird mit einer mikrobiologisch belasteten Flüssigkeit durchströmt. An dieser Elektrodenanordung wird der Detektionsmodus ausgeführt. Dieser umfasst ein Anlegen einer veränderbaren elektrischen Wechselspannung an die Elektrodenanordnung und ein kontinuierliches Messen des komplexen Widerstandes der von der mikrobiologisch belasteten Flüssigkeit durchströmten Elektrodenanordnung.

Es erfolgt ein Registrieren und/oder ein Aufzeichnen der zeitlichen Änderung des komplexen Widerstandes der durchströmten Elektrodenanordnung. Werden in dem angeschlossenen Rohrsystem Desinfektionsmaßnahmen, wie Chlorung, Heisswasser oder Cleaning in Place Prozesse (CIP), durchgeführt, werden analog im Detektionsmodus des Biofilmsensors die Veränderung des Biofilms ermittelt. Damit besteht die Möglichkeit die Reinigungswirkung der durchgeführten Desinfektionsmaßnahme zu ermitteln und gegebenenfalls zu optimieren.

Bei Bedarf kann zeitweise anstelle des Detektionsmodus ein Reinigungsmodus ausgeführt werden. Dabei wird die Elektrodenanordnung mit einer veränderbaren und parametrierbaren Gleichspannung in einer Stärke beaufschlagt, bei der eine Entfernung abgelagerter mikrobiologischer Beläge auf den Oberflächen der Elektrodenanordnung erfolgt. Diese ermöglicht ein Reinigen des Biofilmsensors und eine Rekalibrierung durch eine Wiederherstellung in den Ausgangszustand.

Bei einer Ausführungsform des Verfahrens werden bei dem Ausführen des Detektionsmodus Betrag und/oder Phasenwinkel und/oder der Imaginärteil des komplexen Widerstandes bei einer Frequenz der elektrischen Wechselspannung bestimmt.

Bei einer Ausführungsform des Verfahrens wird bei dem Ausführen des Detektionsmodus der Betrag und/oder Phasenwinkel und/oder der Imaginärteil des komplexen Widerstandes bei mindestens zwei verschiedenen Frequenzen der Wechselspannung oder über ein vorgegebenes durchstimmbares Frequenzintervall als ein Spektrum bestimmt.

Die Amplitude der Wechselspannung im Detektionsmodus ist variabel einstellbar und parametrisierbar. Die Stärke der Gleichspannung im Reinigungsmodus ist ebenfalls variabel einstellbar und parametrisierbar.

Nachfolgend sollen der erfindungsgemäße Biofilmsensor und das mit dem Biofilmsensor ausgeführte Verfahren anhand von Ausführungsbeispielen näher erläutert werden.

Der erfindungsgemäße Biosensor besteht aus einer dichten elektrisch leitfähigen unterstöchiometrischen Rohrkeramik, vorzugsweise Titanoxid oder Titandioxid, in deren Innenraum ein metallisches Rohr oder ein Rohrstab zentriert integriert ist. Das metallische Rohr bzw. der Rohrstab bestehen aus einem korrosionsbeständigen Metall, beispielsweise aus Edelstahl. Im röhrenförmigen Hohlraum der Rohrkeramik, also im Bereich zwischen der inneren Oberfläche der Rohrkeramik und dem metallischen Rohrstab, befindet sich das mikrobiologisch belastete Wasser. Die elektrisch leitende Rohrkeramik ist von außen so kontaktiert, dass ein Messkontakt für elektrische Messungen herausgeführt ist. Der metallische Rohrstab ist ebenfalls aus dem inneren Rohrsystem wasserdicht und elektrisch isolierend zu der Rohrkeramik herausgeführt, sodass eine weitere elektrische Kontaktierung an diesem ermöglicht wird.

Die Enden der Rohrkeramik sind mit Rohradaptern versehen. Über die Rohradapter können Rohre beiderseitig wasserdicht angeschlossen werden, um damit einen kontinuierlichen Durchfluss von Flüssigkeit, d.h. insbesondere Wasser, durch das Innere der Rohrkeramik zu gewährleisten.

Zwischen den beiden Kontakten der Rohrkeramik und dem inneren metallischen Leiter wird ein elektrisches Messinstrument angeschlossen, welches eine kontinuierliche Messung des komplexen Widerstandes zwischen den beiden Rohrwandungen und der Flüssigkeit ermöglicht.

Dieser komplexe elektrische Widerstand ist frequenzabhängig und wird durch einen Real- und Imaginärteil charakterisiert. Der darin enthaltene Realteil spiegelt den ohmschen elektrischen Widerstand und der Imaginärteil den kapazitiven und induktiven Widerstand je Messfrequenz der Anordnung aus dem Keramikrohr, der im Inneren befindlichen Flüssigkeit und dem metallischem Rohrstab wider.

Da elektrisch leitfähige Keramiken, insbesondere auch als Implantate, eine hohe Bioaktivität und Bioverträglichkeit im Vergleich zu Kunststoffen oder metallischen Materialien aufweisen, erfolgt eine sehr frühe Besiedelung der Keramikoberflächen mit einem Biofilm. Dieser aufwachsende Biofilm ist in einem sehr frühen Zustand schon in der Veränderung des komplexen Widerstandes nachzuweisen. Mit zunehmender Biofilmbildung verändert sich auch der komplexe Widerstand des Messsystems. Charakteristisch sind dabei besonders die imaginären Anteile des komplexen Widerstandes, wobei der Leitwert der Flüssigkeit vorwiegend durch den Realteil des komplexen Widerstandes charakterisiert wird.

Entsprechend lässt sich auch eine Veränderung des komplexen Widerstandes nach Durchführung einer Desinfektionsmaßnahme an der zu testenden Flüssigkeit feststellen und registrieren. Ist die durchgeführte Desinfektionsmaßnahme wirkungsvoll, so zeigt sich die Beseitigung des Biofilms im Biofilmsensor darin, dass der Ausgangswert des komplexen Widerstandswertes wieder erreicht wird.

Somit ist die Veränderung des komplexen elektrischen Widerstandes ein Maß dafür, wie sich der auf der Keramik aufgelagerte Biofilm in einem Flüssigkeitssystem verändert.

Da der Aufbau des Biofilmsensors dem eines Rohrkondensators ähnelt, kann mit dieser Anordnung auch zeitweise eine aktive Elektrolyse durchgeführt werden. Dies erfolgt, indem eine Gleichspannung zwischen der Rohrkeramik und dem inneren metallischen Rohrstab angelegt wird. Die Rohrkeramik wird dabei auf ein positives und der innere metallische Rohrstab auf ein negatives Potential gelegt.

Das Ergebnis dieser Elektrolyse ist eine vollständige Entfernung von mikrobiologischen Belegen auf den Oberflächen. Hierdurch kann ein Biosensor wieder auf einen Ausgangszustand gereinigt und somit neu kalibriert werden.

Der Biofilmsensor zeichnet sich also in einem Ausführungsbeispiel dadurch aus, dass dieser einen Rohrkondensator aus zwei Elektroden mit einer wasserdicht elektrisch leitenden Rohrkeramik und mit einem metallischen festen Rohrstab bildet und der Zwischenraum von Flüssigkeiten durchströmt werden kann, indem der sich ergebende veränderliche elektrische komplexe Widerstand zwischen den zwei Elektroden und der Flüssigkeit ein Maß für die veränderliche Biofilmbildung darstellt.

Die elektrisch leitende Rohrkeramik besteht beispielsweise aus wasserdichtem elektrisch leitfähigem Titandioxidmaterial und die metallische Rohrelektrode besteht beispielsweise aus Edelstahl. Das Titandioxidmaterial ist insbesondere unterstöchiometrisch. Das bedeutet, dass im Gitter der Keramik kationische oder anionische Fehlstellen vorhanden sind, die als elektrischer Ladungsträger dienen und somit der Keramik die Leitfähigkeit verleihen.

Der Biofilmsensor kann kalibriert und gereinigt werden, indem zeitweise eine aktive Elektrolyse zwischen den beiden Elektroden betrieben wird. Das Maß der Biofilmveränderung widerspiegelt sich in den frequenzabhängigen kapazitiven und induktiven elektrischen Widerstandsveränderungen.

Der Biofilmsensor und das Verfahren zur Biofilmdetektion sollen nachfolgend anhand beispielhafter Figuren näher beschrieben werden.

Es zeigt:

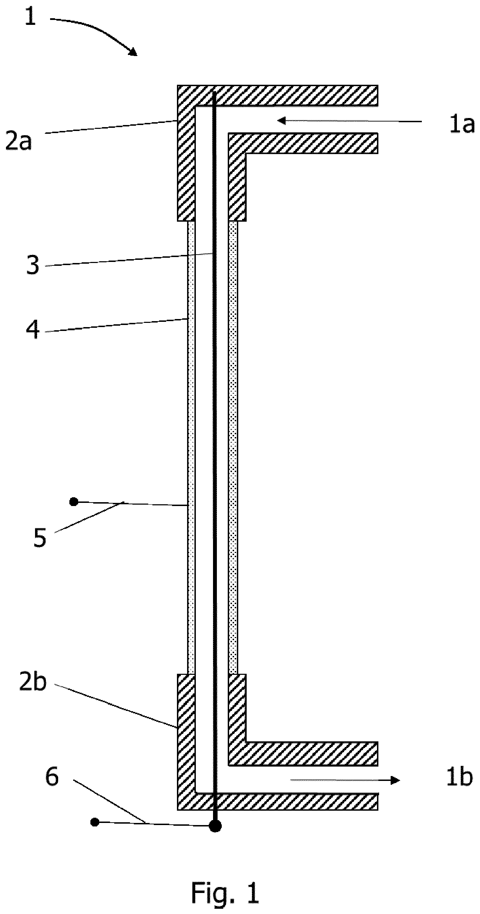

Fig. 1 ein Ausführungsbeispiel des erfindungsgemäßen Biofilmsensors,
Fig. 2 den Biofilmsensor zusammen mit einer Messeinheit und einer Steuereinheit.

Der beispielhafte Biofilmsensor 1 enthält als ein Rohrsystem jeweils einen Probeneingangsanschluss 2a und einem Probenausgangsanschluss 2b, welche einen Einlassstrom 1a und einen Auslassstrom 1b der Flüssigkeit gewährleisten. Der Probeneingangsanschluss 2a und der Probenausgangsanschluss 2b können jeweils als Rohradapter ausgeführt sein. Im Allgemeinen handelt es sich bei den Anschlüssen 2a und 2b jeweils um einen Rohrflanschanschluss für ein Probeneingangsrohr bzw. ein Probenausgangsrohr in und aus dem Biofilmsensor.

Weiterhin ist ein dichtes, elektrisch leitfähiges Keramikrohr als eine Keramikelektrode 4 vorgesehen, welches zwischen den Probeneingangs- und Probenausgangsanschlüssen 2a und 2b drucksicher und wasserdicht montiert. Diese wirkt als elektrisch leitende Keramikelektrode in Rohrform. Bei einer Ausführungsform der Probeneingangs- und -ausgangsanschlüsse als Rohradapter können die Verbindungen zwischen dem Keramikrohr und den Rohradaptern lösbar, beispielsweise als Gummimuffen, ausgeführt sein.

Weiterhin ist im Keramikrohr der Keramikelektrode 4 eine metallische Stabelektrode 3 zentriert positioniert. Die Stabelektrode 3 ist in dem Probeneingangsanschluss 2a und dem Probenausgangsanschluss 2b elektrisch isoliert und drucksicher montiert. Die Stabelektrode 3 ist wasserdicht aus einem der Anschlüsse 2a oder 2b herausgeführt und bildet am Stabende einen elektrischen Anschlusskontakt 6. Außerdem ist an der äußeren Oberfläche der Rohrkeramik der Keramikelektrode 4 ein elektrischer Anschluss 5 realisiert.

Die Stabelektrode 3 kann entweder ein massiver Metallstab oder auch ein Rohrstab sein.

Mit dem Biofilmsensor sind zwei unterschiedliche Betriebsmodi möglich. Die Biofilmdetektion erfolgt in einem Detektionsmodus, die Reinigung des Biofilmsensors erfolgt in einem Reinigungsmodus. Durch den Reinigungsmodus kann der im Biofilmsensor abgelagerte Biofilm beseitigt und der Biofilmsensor auf einem definierten Ausgangszustand rekalibriert werden.

Der Detektionsmodus wird wie folgt ausgeführt. Zwischen den elektrischen Anschlüssen 5 und 6 wird eine elektrische Wechselspannung angelegt. Mittels einer Messanordnung 7 wird der komplexe elektrische Widerstand der Anordnung aus der Keramikelektrode 4, der Stabelektrode 3 und der durchströmenden Flüssigkeit gemessen und über einen im Prinzip beliebig langen Zeitraum hinweg überwacht. Die zeitliche Veränderung des komplexen elektrischen Widerstandes ist dann ein Maß für die Biofilmveränderung auf der inneren Oberfläche der Keramikelektrode, d.h. allgemein im von der Flüssigkeit durchflossenen Röhrenförmigen Hohlraum.

Zur Messung der Biofilmbedeckung auf der Rohrkeramik der Keramikelektrode 4 wird beispielsweise eine sinusförmige Wechselspannung angelegt. Die Amplitude der angelegten Wechselspannung ist zweckmäßigerweise so groß, dass der auf der Rohrkeramik abgelagerte Biofilm durch die Messung möglichst wenig beeinflusst wird. Sie beträgt beispielsweise 20 bis 50 mV. Die Amplitude ist variierbar und parametrisierbar. Sie kann insbesondere in einem Messprogramm verändert werden.

In Verbindung damit wird der komplexe elektrische Widerstand der Elektrodenanordnung aus dem Keramikrohr der Keramikelektrode 4, der Stabelektrode 3 und der zwischen beiden Elektroden befindlichen Flüssigkeit, d.h. insbesondere Wasser, gemessen. Der komplexe elektrische Widerstand kann dabei sowohl bei einer fest eingestellten Frequenz als auch in Abhängigkeit mehrerer einzelner Frequenzen der angelegten Wechselspannung, aber auch in Form eines quasi kontinuierlichen Spektrums, bestimmt werden. Bei der Erfassung des Spektrums wird der jeweils zu erfassende Frequenzbereich der Wechselspannung durchgestimmt und dabei der komplexe elektrische Widerstand in Abhängigkeit von der Wechselspannungsfrequenz erfasst. Diese Erfassung des Spektrums wird zeitlich in regelmäßigen Abständen wiederholt, sodass im Ergebnis der komplexe elektrische Widerstand über der Frequenz und der Zeit zweidimensional aufgezeichnet wird.

Aus den gemessenen Werten des komplexen elektrischen Widerstandes können sodann dessen Realteil, der Imaginärteil, der Betrag und/oder auch der Phasenwinkel in der komplexen Widerstandsebene ermittelt werden. In der Regel enthält der Realteil des komplexen Widerstands vor allem Informationen über den ohmschen Widerstand der Flüssigkeit als solcher und wird von dem wachsenden Biofilm kaum beeinflusst. Der wachsende Biofilm macht sich durch zusätzliche kapazitive oder induktive Anteile in der Elektrodenkonfiguration des Biofilmsensors bemerkbar, die zu frequenzabhängigen Anteilen im Imaginärteil des komplexen Widerstands führen. Das Wachstum des Biofilms äußert sich somit somit vor allem in einer Veränderung im Imaginärteil des komplexen Widerstands.

Maßgebliche Messgrößen sind sowohl der Betrag des komplexen Widerstands, also der Scheinwiderstand, als auch gegebenenfalls dessen Phasenwinkel und somit auch der Realteil und der Imaginärteil des komplexen Widerstands als dessen vektorielle Komponenten.

Weiterhin kann eine Kalibrierung und gezielte bakteriologische Reinigung des Biofilmsensors erfolgen. Dies erfolgt im Reinigungsmodus, der wie folgt ausgeführt wird.

Im Reinigungsmodus wird eine aktive Gleichspannung an die elektrischen Anschlüsse 5 und 6 der Keramikelektrode 4 bzw. der Stabelektrode 3 angelegt. Dabei wird insbesondere ein positives Potential an dem elektrischen Anschluss 5 und ein negatives Potential an den Anschluss 6 so angelegt, dass eine Elektrolyse zwischen der Stabelektrode 3 und der Keramikelektrode 4 realisiert werden kann. Die angelegte Gleichspannung dient der bakteriologischen Reinigung und damit zur Beseitigung des Biofilms und zur Rekalibrierung des Biofilmsensors. Sie ist zweckmäßigerweise auf eine wirkungsvolle Zerstörung des abgelagerten Biofilms gerichtet. Die Stärke der Gleichspannung beträgt 10 bis 100 V, beispielsweise 30 V. Sie ist insbesondere variabel einstellbar und kann innerhalb eines Reinigungsprogramm auch parametrisierbar sein. Somit können auch in aufeinander folgenden Zeitintervallen verschiedene Gleichspannungswerte an den Biofilmsensor angelegt werden.

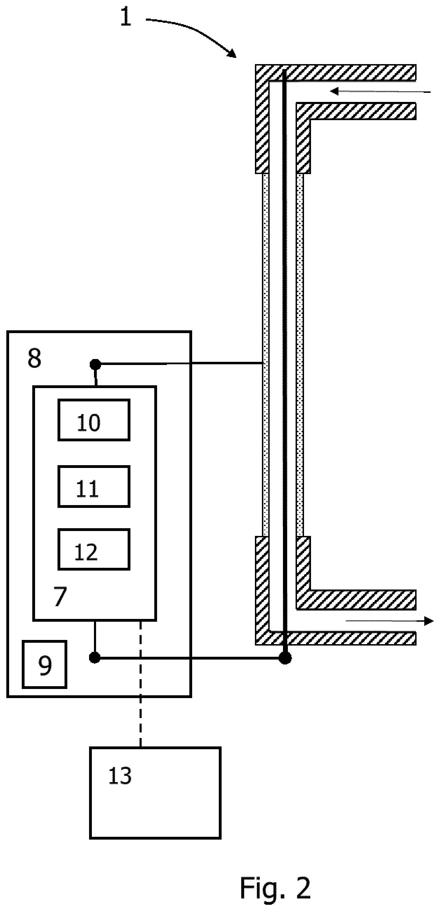

Fig. 2 zeigt eine beispielhafte Anordnung aus dem Biofilmsensor 1 und einer Steuereinheit 8 zum Betreiben des Biofilmsensors mit einer Messanordnung 7. Die Messanordnung 7 ist im hier vorliegenden Beispiel ein Bestandteil der Steuereinheit 8. Die Steuereinheit 8 ermöglicht zum einen den erwähnten Detektionsmodus, bei dem die Messanordnung 7 zur Anwendung kommt, und zum anderen den Reinigungsmodus, bei dem die Messanordnung 7 inaktiv ist und stattdessen der Biofilmsensor 1 mit einer Gleichspannung aus einer Gleichspannungsquelle 9 beaufschlagt wird, um den gebildeten Biofilm auf der Keramikelektrode zu beseitigen.

Die Messanordnung 7 enthält einen Frequenzgenerator 10 zum Erzeugen einer veränderlichen Wechselspannung. Der Frequenzgenerator ist entweder auf eine einzelne Frequenz eingerichtet, er kann aber auch mehrere Frequenzen erzeugen oder auch innerhalb eines bestimmten Frequenzbereichs durchstimmbar sein. Die Amplitude der Wechselspannung ist variabel einstellbar und parametrisierbar. Die dabei erzeugten periodischen Spannungsverläufe sind nicht von vornherein auf eine bestimmte Form beschränkt, sie können beispielsweise sinusartig, sägezahnförmig oder rechteckförmig sein.

Die Messanordnung 7 enthält außerdem eine Messschaltung 11 zum Bestimmen des komplexen elektrischen Widerstandes der flüssigkeitsgefüllten Elektrodenanordnung des Biofilmsensors und eine Auswerteeinheit 12 zum Errechnen des Betrages, des Imaginärteiles des komplexen Widerstandes, des Phasenwinkels und/oder des Realteiles des komplexen Widerstandes.

Eine Speichereinheit 13 zeichnet die gemessenen und nachträglich ermittelten Werte des komplexen Widerstandes und der daraus abgeleiteten Größen auf und ermöglicht so deren zeitliche Verfolgung. Die Speichereinheit 13 kann bezüglich der Steuereinheit 8 intern oder extern als entferntes Gerät angeordnet sein. Maßgeblich für die gespeicherten Werte sind in erster Linie zeitliche Veränderungen der gemessenen Werte des komplexen elektrischen Widerstands sowie der danach abgeleiteten genannten Parameter. Zeitlich konstante Werte zeigen dabei einen entweder nicht vorhandenen oder in seiner Dicke und Ausdehnung unveränderten Biofilm an, sich ändernde Werte einen sich entsprechend ändernden, in der Regel ausdehnenden Biofilm. Eine Rückveränderung der geänderten Werte infolge einer Wasserbehandlung, wie beispielsweise einer Chlorierung, weist auf eine Zurückdrängung des Biofilms bzw. dessen teilweise oder vollständige Beseitigung hin. Die Speichereinheit 13 kann hierzu zusätzlich die Eingabe von Ereignissen in Bezug auf Desinfektionsmaßnahmen, Wasserbehandlungen und dergleichen Eingriffe ermöglichen, sodass den entsprechenden Messwertveränderungen ein entsprechendes Ereignis zugewiesen und im Messsystem zeitlich protokolliert werden kann.

Das Zurücksetzen des Biofilmsensors auf einen biofilmfreien Zustand zum Zweck einer Rekalibrierung ist jederzeit wie beschrieben durch den Reinigungsmodus und durch das Aktivieren der Gleichspannungsquelle 9 möglich. Die Gleichspannungsquelle liefert eine Gleichspannung, die variierbar und parametrisierbar ist.

Hierzu bietet die Steuereinheit ein entsprechendes Umschaltmittel, beispielsweise einen Kippschalter oder Taster.

Der Biofilmsensor und das Verfahren zur Biofilmdetektion wurden anhand beispielhafter Ausführungsformen erläutert. Weitere Ausführungsformen ergeben sich aus den Unteransprüchen.

### Bezugszeichenliste

- 1: Biofilmsensor
- 1a: Eintritt Flüssigkeitstrom in den Biofilmsensor
- 1b: Austritt Flüssigkeitstrom aus dem Biofilmsensor
- 2a: Probeneingangsanschluss,
- 2b: Probenausgangsanschluss
- 3: Stabelektrode
- 4: Keramikelektrode
- 5: Elektrischer Anschluss für Keramikelektrode
- 6: Elektrischer Anschluss für metallische Stabelektrode
- 7: Messanordnung
- 8: Steuereinheit
- 9: Gleichspannungsquelle
- 10: Frequenzgenerator
- 11: Messschaltung
- 12: Auswerteeinheit
- 13: Speichereinheit

## Patentansprüche

1. Biofilmsensor (1) mit einer Elektrodenanordnung und einer schaltbaren Messanordnung (7),
wobei die Elektrodenanordnung eine mit einer Flüssigkeit durchströmbaren elektrisch kontaktierten rohrförmigen Keramikelektrode (4) aus einem elektrisch leitfähigen Keramikmaterial mit einem an einen an der rohrförmigen Keramikelektrode angeordneten Probeneingangsanschluss (2a) für ein Probeneingangsrohr und einen Probenausgangsanschluss (2b) für ein Probenausgangsrohr und einer im Lumen, d.h. im röhrenförmigen Hohlraum der Keramikelektrode (4) verlaufenden elektrisch kontaktierten Stabelektrode (3) umfasst,
wobei der Probeneingangsanschluss (2a) und der Probenausgangsanschluss (2b) an gegenüberliegenden Enden des Lumens der rohrförmigen Keramikelektrode (4) angeordnet sind,
wobei die schaltbare Messanordnung (7) dazu eingerichtet ist in einem Detektionsmodus an der Elektrodenanordnung eine veränderbare elektrische Wechselspannung anzulegen und zwischen der Keramikelektrode (4) und der Stabelektrode (3) einen frequenzabhängigen komplexen elektrischen Widerstandes der mit der Flüssigkeit durchströmten Elektrodenanordnung zu bestimmen.

2. Biofilmsensor (1) nach Anspruch 1,
**dadurch gekennzeichnet, dass**
das elektrisch leitfähige Keramikmaterial der rohrförmigen Keramikelektrode (4) ein wasserdichtes und druckbeständiges elektrisch leitfähiges unterstöchiometrisches Titandioxidmaterial ist, bei dem im Gitter der Keramik kationische oder anionische Fehlstellen vorhanden sind, die als elektrische Ladungsträger dienen.

3. Biofilmsensor nach einem der Ansprüche 1 oder 2,
**dadurch gekennzeichnet, dass**
die rohrförmige Keramikelektrode (4) elektrisch isolierend bezüglich der inneren Stabelektrode (3) montiert und kontaktiert ist.

4. Biofilmsensor (1) nach Anspruch 1 oder 3,
**dadurch gekennzeichnet, dass**
die Stabelektrode (3) aus einem korrosionsbeständigen metallischen Material, insbesondere aus einem Edelstahl, besteht.

5. Biofilmsensor (1) nach einem der Ansprüche 1 bis 4,
**gekennzeichnet durch**
eine zwischen die Keramikelektrode (4) und die Stabelektrode (3) angeordnete variable Gleichspannungsquelle (9) zum Anlegen einer veränderlich parametrisierbaren Gleichspannung, zum Ausführen einer Elektrolyse für ein Reduzieren und Beseitigen eines Biofilms in der Elektrodenanordnung.

6. Verfahren zur Biofilmdetektion in einer flüssigkeitsgefüllten Umgebung in einem Detektionsmodus und mit folgende Verfahrensschritte:
- Verwenden eines Biofilmsensors (1) mit einer schaltbaren Messanordnung (7) und einer Elektrodenanordnung aus einer rohrförmigen Keramikelektrode (4) und einer im Lumen, d.h. im röhrenförmigen Hohlraum der Keramikelektrode, angeordneten Stabelektrode (3),
- Durchströmen der Elektrodenanordnung von einem Probeneingangsanschluss (2a) zu einem Probenausgangsanschluss (2b) am gegenüberliegenden Enden des Lumens der rohrförmigen Keramikelektrode (4) mit einer mikrobiologisch belasteten Flüssigkeit,
- Ausführen des Detektionsmodus durch die schaltbaren Messanordnung (7), umfassend ein Anlegen einer veränderbaren elektrischen Wechselspannung an die Elektrodenanordnung und kontinuierliches Messen des komplexen Widerstandes der von der mikrobiologisch belasteten Flüssigkeit durchströmten Elektrodenanordnung,
- Registrieren und/oder Aufzeichnen der zeitlichen Änderung des komplexen Widerstandes der durchströmten Elektrodenanordnung.

7. Verfahren nach Anspruch 6,
**dadurch gekennzeichnet, dass**
zeitweise ein Reinigungsmodus ausgeführt werden kann, wobei die Elektrodenanordnung mit einer veränderbaren Gleichspannung in einer Stärke beaufschlagt wird, bei der eine Reduzierung bzw. Entfernung abgelagerter mikrobiologischer Beläge auf den Oberflächen der Elektrodenanordnung erfolgt.

8. Verfahren nach Anspruch 7,
**dadurch gekennzeichnet, dass**
bei dem Ausführen des Detektionsmodus der Betrag und/oder der Phasenwinkel und/oder der Imaginärteil des komplexen Widerstandes bei einer Frequenz der elektrischen Wechselspannung bestimmt wird.

9. Verfahren nach Anspruch 8,
**dadurch gekennzeichnet, dass**
bei Ausführen des Detektionsmodus Betrag und/oder Phasenwinkel und/oder der Imaginärteil des komplexen Widerstandes bei mindestens zwei verschiedenen Frequenzen der Wechselspannung oder über ein vorgegebenes durchstimmbares Frequenzintervall als ein Spektrum bestimmt wird.

10. Verfahren nach einem der Ansprüche 6, 8 oder 9,
**dadurch gekennzeichnet, dass**
die Amplitude und/oder die Frequenz der Wechselspannung variabel einstellbar und parametrisierbar ist.

11. Verfahren nach Anspruch 7,
**dadurch gekennzeichnet, dass**
die Stärke der Gleichspannung variabel einstellbar und parametrisierbar ist.

## Claims

1. Biofilm sensor (1) having an electrode assembly and a switchable measurement assembly (7),
wherein the electrode assembly
comprises an electrically contacted tubular ceramic electrode (4), through which a liquid can flow, made of an electrically conductive ceramic material having a sample inlet fitting (2a), arranged on the tubular ceramic electrode, for a sample inlet tube and a sample outlet fitting (2b) for a sample outlet tube and an electrically contacted bar electrode (3) extending in the lumen, i.e. in the tubular cavity of the ceramic electrode (4),
wherein the sample inlet fitting (2a) and the sample outlet fitting (2b) are arranged at opposite ends of the lumen of the tubular ceramic electrode (4),
wherein the switchable measurement assembly (7) is configured, in a detection mode, to apply a variable electrical AC voltage to the electrode assembly and to determine, between the ceramic electrode (4) and the bar electrode (3), a frequency-dependent complex electrical resistance of the electrode assembly through which the liquid flows.

2. Biofilm sensor (1) as claimed in claim 1,
**characterized in that**
the electrically conductive ceramic material of the tubular ceramic electrode (4) is a watertight and pressure-resistant electrically conductive substoichio-metric titanium dioxide material, in which cationic or anionic flaws, which serve as electric charge carriers, are present in the lattice of the ceramic.

3. Biofilm sensor as claimed in either of claims 1 or 2,
**characterized in that**
the tubular ceramic electrode (4) is installed and contacted in an electrically insulated manner with respect to the inner bar electrode (3).

4. Biofilm sensor (1) as claimed in claim 1 or 3,
**characterized in that**
the bar electrode (3) consists of a corrosion-resistant metallic material, in particular of a stainless steel.

5. Biofilm sensor (1) as claimed in any one of claims 1 to 4,
**characterized by**
a variable DC voltage source (9) arranged between the ceramic electrode (4) and the bar electrode (3) for applying a variable parameterizable DC voltage, for executing an electrolysis to reduce and eliminate a biofilm in the electrode assembly.

6. Method for biofilm detection in a liquid-filled environment in a detection mode and comprising the following method steps:
- using a biofilm sensor (1) having a switchable measurement assembly (7) and electrode assembly made up of a tubular ceramic electrode (4) and a bar electrode (3) arranged in the lumen, i.e. in the tubular cavity of the ceramic electrode,
- causing a microbiologically loaded liquid to flow through the electrode assembly from a sample inlet fitting (2a) to a sample outlet fitting (2b) at opposite ends of the lumen of the tubular ceramic electrode (4),
- executing the detection mode by way of the switchable measurement assembly (7), comprising applying a variable electrical AC voltage to the electrode assembly and continuously measuring the complex resistance of the electrode assembly through which the microbiologically loaded liquid flows,
- registering and/or recording the change over time of the complex resistance of the electrode assembly through which flow occurs.

7. Method as claimed in claim 6,
**characterized in that**
sometimes a cleaning mode can be executed, wherein the electrode assembly is subjected to a variable DC voltage at a strength at which a reduction or removal of deposited microbiological coatings on the surfaces of the electrode assembly takes place.

8. Method as claimed in claim 7,
**characterized in that**
upon the execution of the detection mode, the absolute value and/or the phase angle and/or the imaginary part of the complex resistance is determined at a frequency of the electrical AC voltage.

9. Method as claimed in claim 8,
**characterized in that**
upon the execution of the detection mode, the absolute value and/or the phase angle and/or the imaginary part of the complex resistance is determined at at least two different frequencies of the AC voltage or across a predetermined tunable frequency interval as a spectrum.

10. Method as claimed in any one of claims 6, 8, or 9,
**characterized in that**
the amplitude and/or the frequency of the AC voltage is variably settable and parameterizable.

11. Method as claimed in claim 7,
**characterized in that**
the strength of the DC voltage is variably settable and parameterizable.

## Revendications

1. Détecteur de biofilm (1) muni d'une disposition d'électrodes et d'un dispositif de mesure commutable (7),
dans lequel la disposition d'électrodes comprend une électrode en céramique tubulaire (4) pouvant être parcourue par un liquide et mise en contact électrique, faite d'un matériau céramique conducteur électrique, avec un raccord d'entrée d'échantillon (2a) disposé sur l'électrode en céramique tubulaire pour un tuyau d'entrée d'échantillon et un raccord de sortie d'échantillon (2b) pour un tuyau de sortie d'échantillon et une électrode en baguette (3) mise en contact électrique et passant dans la lumière, c'est-à-dire dans la cavité tubulaire de l'électrode en céramique (4),
dans lequel le raccord d'entrée d'échantillon (2a) et le raccord de sortie d'échantillon (2b) sont disposés à des extrémités opposées de la lumière de l'électrode en céramique tubulaire (4),
dans lequel le dispositif de mesure commutable (7) est configuré pour appliquer à la disposition d'électrodes, dans un mode de détection, un courant alternatif électrique modifiable et pour déterminer entre l'électrode en céramique (4) et l'électrode en baguette (3) une résistance électrique complexe, dépendante de la fréquence, de la disposition d'électrodes parcourue par le liquide.

2. Détecteur de biofilm (1) selon la revendication 1, **caractérisé en ce que** le matériau céramique conducteur électrique de l'électrode en céramique tubulaire (4) est un matériau à base de dioxyde de titane conducteur électrique, étanche à l'eau et résistant à la pression, en proportion sous-stœchiométrique, dans lequel le réseau cristallin de la céramique contient des défauts cationiques ou anioniques qui servent de porteurs de charge électriques.

3. Détecteur de biofilm selon l'une des revendications 1 ou 2, **caractérisé en ce que** l'électrode en céramique tubulaire (4) est montée et mise en contact de façon isolante électrique par rapport à l'électrode en baguette interne (3).

4. Détecteur de biofilm (1) selon la revendication 1 ou 3, **caractérisé en ce que** l'électrode en baguette (3) se compose d'un matériau métallique résistant à la corrosion, en particulier d'un acier inoxydable.

5. Détecteur de biofilm (1) selon l'une des revendications 1 à 4, **caractérisé en ce qu'**il comprend une source de courant continu variable (9) disposée entre l'électrode en céramique (4) et l'électrode en baguette (3) pour appliquer un courant continu paramétrable de façon modifiable afin de réaliser une électrolyse en vue de la réduction et de l'élimination d'un biofilm dans la disposition d'électrodes.

6. Procédé pour la détection de biofilm dans un environnement rempli de liquide dans un mode de détection, comprenant les étapes de procédé suivantes :
- utilisation d'un détecteur de biofilm (1) muni d'un dispositif de mesure commutable (7) et d'une disposition d'électrodes composée d'une électrode en céramique tubulaire (4) et d'une électrode en baguette (3) disposée dans la lumière, c'est-à-dire dans la cavité tubulaire de l'électrode en céramique,
- écoulement d'un liquide chargé de micro-organismes à travers la disposition d'électrodes, d'un raccord d'entrée d'échantillon (2a) à un raccord de sortie d'échantillon (2b) situés à des extrémités opposées de la lumière de l'électrode en céramique tubulaire (4),
- exécution du mode de détection par le dispositif de mesure commutable (7), comprenant une application d'un courant alternatif électrique modifiable à la disposition d'électrodes et une mesure en continu de la résistance complexe de la disposition d'électrodes parcourue par le liquide chargé de micro-organismes,
- enregistrement et/ou relevé de l'évolution dans le temps de la résistance complexe de la disposition d'électrodes parcourue par le liquide.

7. Procédé selon la revendication 6, **caractérisé en ce qu'**un mode de nettoyage peut être exécuté temporairement, dans lequel la disposition d'électrodes est soumise à un courant continu modifiable d'une intensité telle qu'il se produit une réduction et une élimination de couches microbiennes déposées sur les surfaces de la disposition d'électrodes.

8. Procédé selon la revendication 7, **caractérisé en ce que**, lors de l'exécution du mode de détection, la grandeur et/ou l'angle de phase et/ou la partie imaginaire de la résistance complexe sont déterminés à une fréquence du courant alternatif électrique.

9. Procédé selon la revendication 8, **caractérisé en ce que**, lors de l'exécution du mode de détection, la grandeur et/ou l'angle de phase et/ou la partie imaginaire de la résistance complexe sont déterminés à au moins deux fréquences différentes du courant alternatif ou sur un intervalle de fréquence variable prédéterminé formant un spectre.

10. Procédé selon l'une des revendications 6, 8 ou 9, **caractérisé en ce que** l'amplitude et/ou la fréquence du courant alternatif sont réglables et paramétrables de façon variable.

11. Procédé selon la revendication 7, **caractérisé en ce que** l'intensité du courant continu est réglable et paramétrable de façon variable.
